# EUROPEAN PATENT APPLICATION

(11) **EP 4 527 417 A1**
(43) Date of publication of application: **26.03.2025**
(21) Application number: 23807681.4
(22) Date of filing: 17.05.2023
(51) Int. Cl.: A61K 48/00, A61P 35/00, A61P 35/04, A61P 37/04, C12N 15/11, A61K 31/7088, A61K 31/712

(54) **ANTI-TUMOR AGENT CONTAINING RNA, AND USE THEREOF**

(30) Priority: 17.05.2022 JP 2022081120
(71) Applicant: GF Mille Co. Ltd., Gifu-shi, Gifu 501-1193 (JP); Shinshu University, Matsumoto-shi, Nagano 390-8621 (JP); NATIONAL UNIVERSITY CORPORATION TOKAI NATIONAL HIGHER EDUCATION AND RESEARCH SYSTEM, Nagoya-shi, Aichi 464-8601 (JP)
(72) Inventor: HIRATSUKA Sachie, Matsumoto-shi, Nagano 390-8621 (JP); TOMITA Takeshi, Matsumoto-shi, Nagano 390-8621 (JP); HAYASHI Hikaru, Matsumoto-shi, Nagano 390-8621 (JP); UENO Yoshihito, Gifu-shi, Gifu 501-1193 (JP); CHANO Tokuhiro, Gifu-shi, Gifu 501-1193 (JP); FURUICHI Yasuhiro, Gifu-shi, Gifu 501-1193 (JP); KAWADE Miwa, Gifu-shi, Gifu 501-1193 (JP); KAKIZAWA Yuri, Gifu-shi, Gifu 501-1193 (JP)
(74) Representative: Leonard, Thomas Charles
(86) International application number: PCT/JP2023/018498
(87) International publication number: WO 2023/224080

(57) **Abstract**

An antitumor agent containing, as an active ingredient, RNA having a base sequence selected from the group consisting of (1) and (2) below, and having at least one nucleotide unit represented by Formula (1) below in a 3' end region within 20 bases of the 3' end thereof and/or in a 5' end region within 25 bases of the 5' end thereof.
(1) A base sequence represented by SEQ ID NO: 2 or a base sequence having 90% or more identity with such sequence
(2) A base sequence consisting of between 20 and 200 consecutive nucleotides of the base sequence described in (1) above 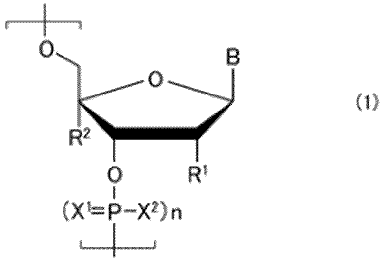 (In Formula (1), R¹ represents a hydroxyl group, a hydroxyl group with a hydrogen atom substituted with an alkyl or alkenyl group, or a halogen atom; R² represents NHR³ with a linking group, the linking group being a divalent hydrocarbon group with one or more carbon atoms; R³ represents a hydrogen atom, an alkyl group, or an alkenyl group; n is 0 or 1; X¹ represents an oxygen atom or sulfur atom; X² represents OH (or O⁻) or SH (or S⁻); and B represents a purine base or a pyrimidine base.)

## Description

### REFERENCE TO RELATED APPLICATIONS

The present application is an application claiming priority based on Japanese Patent Application No. 2022-81120, filed with the Japan Patent Office on May 17, 2022, and the contents thereof being incorporated herein by reference as though forming a part of the present specification. The present specification relates to the use of antitumor agents such as those inhibiting metastasis of tumors containing RNA and methods for activating immune cells, etc.

### BACKGROUND OF THE INVENTION

Potential anti-metastatic immune cells have been discovered recently. Specifically, B220⁺CD11c⁺NK1.1⁺ natural killer (NK) cells have been found to migrate from the liver to the lungs of mice with cancer (Non-Patent Document 1). These NK cells accumulate at fibrinogen⁺ hyperpermeability sites, remove concentrated fibrinogen, and have tumor-destroying potential via interferon gamma (IFNγ) production (Non-Patent Document 2).

In a comparative gene expression analysis between B220⁺CD11c⁺NK1.1⁺NK cells in the liver and B220⁺CD11c⁺NK1.1⁺ cells in the lungs showed that ZC3H12D, which belongs to the RNA-binding zinc finger family, showed the highest rate of change in lung cells. ZC3H12D has been reported as a potential tumor suppressor in lymphoma and lung cancer patients (Non-Patent Document 3, 4).

### CITATION LIST

### NON-PATENT DOCUMENTS

NON-PATENT DOCUMENT 1: EMBO Mol Med 10, doi: 10.15252/emmm.201708643 (2018)
NON-PATENT DOCUMENT 2: Nat Commun 4, 1853, doi: 10.1038/ncomms2856 (2013)
NON-PATENT DOCUMENT 3: Cancer Res 67, 93-99, doi: 10.1158/0008-5472.CAN-06-2723 (2007)
NON-PATENT DOCUMENT 4: Br J Haematol 139, 161-163, doi: 10.1111/j.1365-2141.2007.06752.x (2007)

### SUMMARY OF THE INVENTION

The present inventors, in studies, have suggested that the ZC3H12D protein interacts with extracellular RNA (exRNA) in an anti-metastatic function and activates NK cells.

The present specification provides an antitumor agent with RNA as an active ingredient that is able to effectively interact with ZC3H12D protein to activate NK cells, and uses thereof.
[1] An antitumor agent containing, as an active ingredient, RNA having a base sequence selected from the group consisting of (1) and (2) below, and having at least one first nucleotide unit represented by Formula (1) below or at least one second nucleotide unit represented by Formula (2) below in a 3' end region within 20 bases of the 3' end thereof and/or in a 5' end region within 25 bases of the 5' end thereof.
   (1) A base sequence represented by SEQ ID NO: 2 or a base sequence having 90% or more identity with such sequence
   (2) A base sequence consisting of between 20 and 200 consecutive nucleotides of the base sequence described in (1) above 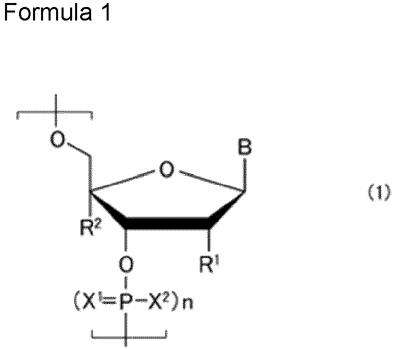 (In Formula (1), R¹ represents a hydroxyl group, a hydroxyl group with a hydrogen atom substituted with an alkyl or alkenyl group, or a halogen atom; R² represents NHR³ with a linking group, the linking group being a divalent hydrocarbon group with one or more carbon atoms; R³ represents a hydrogen atom, an alkyl group, or an alkenyl group; n is 0 or 1; X¹ represents an oxygen atom or sulfur atom; X² represents OH (or O⁻) or SH (or S-); and B represents a purine base or a pyrimidine base.) 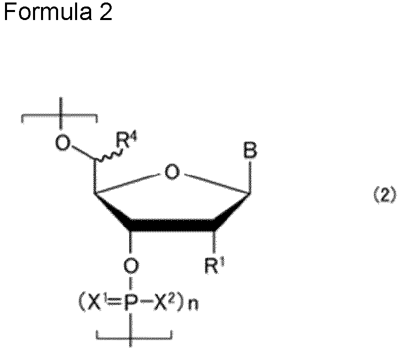 (In Formula (2), R¹ represents a hydroxyl group, a hydroxyl group with a hydrogen atom substituted with an alkyl or alkenyl group, or a halogen atom; R⁴ represents NHR⁵ with a linking group, the linking group being a divalent hydrocarbon group with one or more carbon atoms; R⁵ represents a hydrogen atom, an alkyl group, or an alkenyl group; n is 0 or 1; X¹ represents an oxygen atom or sulfur atom; X² represents OH (or O⁻) or SH (or S⁻); and B represents a purine base or a pyrimidine base.)
[2] The antitumor agent according to [1], wherein the RNA has between 1 and 20 of these nucleotide units per region in the 3' end region and/or 5' end region.
[3] The antitumor agent according to [1] or [2], wherein the first nucleotide unit or second nucleotide unit is a nucleotide unit wherein R¹ is a methoxy group or a fluorine atom and R² is an aminoethyl group in Formula (1) above.
[4] The antitumor agent according to [1] or [2], wherein the first nucleotide unit is a nucleotide unit in which n is 1, R¹ is a fluorine atom, and R² is an aminoethyl group, and the antitumor agent has at least one first nucleotide unit within two bases from the 3' end of the 3' end region and at least one first nucleotide unit within two bases from the 5' end of the 5' end region.
[5] The antitumor agent according to [1] or [2], wherein the second nucleotide unit is a nucleotide unit in which n is 1, R¹ is a methoxy group, and R⁴ is an aminopropyl group, and the antitumor agent has at least one second nucleotide unit within two bases from the 3' end of the 3' end region and at least one second nucleotide unit within two bases from the 5' end of the 5' end region.
[6] The antitumor agent according to [4] or [5], wherein the base sequence has 90% or more identity with between 30 and 70 consecutive bases of the base sequence represented by SEQ ID NO: 4.
[7] The antitumor agent according to [4] or [5], wherein the base sequence has 90% or more identity with between 40 and 60 consecutive bases of the base sequence represented by SEQ ID NO: 4.
[8] The antitumor agent according to [6] or [7], wherein the base sequence is a base sequence having 90% or more identity with the base sequence represented by SEQ ID NOS: 6 or 10.
[9] The antitumor agent according to [8], wherein a base in the first or second nucleotide unit is cytosine or uracil.
[10] The antitumor agent according to [1] to [9], wherein n is 1 in the first or second nucleotide unit, the RNA has a first or second nucleotide unit in the 3' end region and/or 5' end region in which either X¹ or X² in Formula (1) above is S or SH(S⁻).
[11] The antitumor agent according to [1] to [10], wherein at least 80% of the total number of uridine nucleosides in the RNA contain uridine analogues are selected from the group consisting of pseudouridine, 1-methylpseudouridine, 1-ethylpseudouridine, 2-thiouridine, 4'-thiouridine, 5-methylcytosine, 2-thio-1-methyl-1-deaza-pseudouridine, 2-thio-1-methylpseudouridine, 2-thio-5-aza-uridine, 2-thio-dihydropseudouridine, 2-thio-dihydropseudouridine, 2-thio-pseudouridine, 4-methoxy-2-thio-pseudouridine, 4-methoxy-pseudouridine, 4-thio-1-methyl-pseudouridine, 4-thio-pseudouridine, 5-aza-uridine, dihydropseudouridine, 5-methoxyuridine, and 2'-O-methyluridine.
[12] The antitumor agent according to [1] to [11], wherein the RNA is a variant of RNA having the base sequence represented by SEQ ID NO: 1, and the coding sequence from the 88th to 897th base in the base sequence represented by SEQ ID NO: 1 has bases substituted to have 1 to 5 additional stop codons compared to the natural coding sequence.
[13] The antitumor agent according to [1] to [12], wherein the length of the RNA is 20 to 100 bases long.
[14] An immunostimulant containing, as an active ingredient, RNA having a base sequence selected from the group consisting of (1) and (2) below, and having at least one first nucleotide unit represented by Formula (1) below or at least one second nucleotide unit represented by Formula (2) below in a 3' end region within 20 bases of the 3' end thereof and/or in a 5' end region within 25 bases of the 5' end thereof.
   (1) A base sequence represented by SEQ ID NO: 2 or a base sequence having 90% or more identity with such sequence
   (2) A base sequence consisting of between 20 and 200 consecutive nucleotides of the base sequence described in (1) above 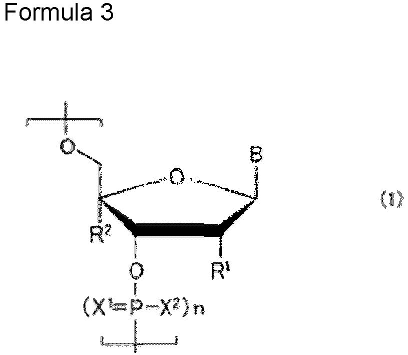 (In Formula (1), R¹ represents a hydroxyl group, a hydroxyl group with a hydrogen atom substituted with an alkyl or alkenyl group, or a halogen atom; R² represents NHR³ with a linking group, the linking group being a divalent hydrocarbon group with one or more carbon atoms; R³ represents a hydrogen atom, an alkyl group, or an alkenyl group; n is 0 or 1; X¹ represents an oxygen atom or sulfur atom; X² represents OH (or O⁻) or SH (or S⁻); and B represents a purine base or a pyrimidine base.) 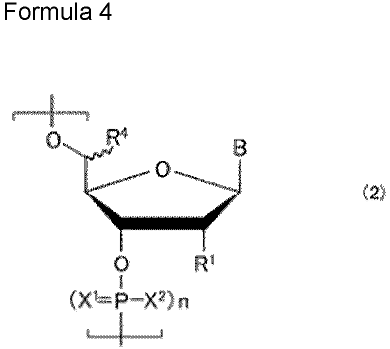 (In Formula (2), R¹ represents a hydroxyl group, a hydroxyl group with a hydrogen atom substituted with an alkyl or alkenyl group, or a halogen atom; R⁴ represents NHR⁵ with a linking group, the linking group being a divalent hydrocarbon group with one or more carbon atoms; R⁵ represents a hydrogen atom, an alkyl group, or an alkenyl group; n is 0 or 1; X¹ represents an oxygen atom or sulfur atom; X² represents OH (or O⁻) or SH (or S⁻); and B represents a purine base or a pyrimidine base.)
[15] A tumor metastasis inhibitor containing, as an active ingredient, RNA having a base sequence selected from the group consisting of (1) and (2) below, and having at least one first nucleotide unit represented by Formula (1) below or at least one second nucleotide unit represented by Formula (2) below in a 3' end region within 20 bases of the 3' end thereof and/or in a 5' end region within 25 bases of the 5' end thereof.
   (1) A base sequence represented by SEQ ID NO: 2 or a base sequence having 90% or more identity with such sequence
   (2) A base sequence consisting of between 20 and 200 consecutive nucleotides of the base sequence described in (1) above 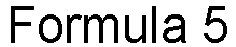 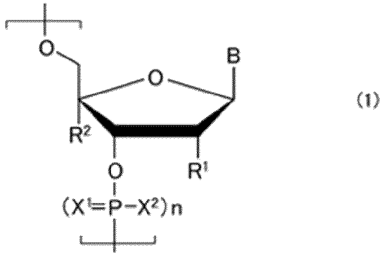 (In Formula (1), R¹ represents a hydroxyl group, a hydroxyl group with a hydrogen atom substituted with an alkyl or alkenyl group, or a halogen atom; R² represents NHR³ with a linking group, the linking group being a divalent hydrocarbon group with one or more carbon atoms; R³ represents a hydrogen atom, an alkyl group, or an alkenyl group; n is 0 or 1; X¹ represents an oxygen atom or sulfur atom; X² represents OH (or O⁻) or SH (or S⁻); and B represents a purine base or a pyrimidine base.) 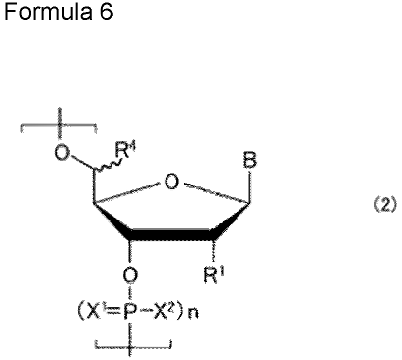 (In Formula (2), R¹ represents a hydroxyl group, a hydroxyl group with a hydrogen atom substituted with an alkyl or alkenyl group, or a halogen atom; R⁴ represents NHR⁵ with a linking group, the linking group being a divalent hydrocarbon group with one or more carbon atoms; R⁵ represents a hydrogen atom, an alkyl group, or an alkenyl group; n is 0 or 1; X¹ represents an oxygen atom or sulfur atom; X² represents OH (or O⁻) or SH (or S⁻); and B represents a purine base or a pyrimidine base.)
[16] An enhancer of an immunostimulatory effect of an immunostimulant or an antitumor effect of an antitumor agent, the enhancer containing, as an active ingredient, RNA having a base sequence selected from the group consisting of (1) and (2) below, and having at least one first nucleotide unit represented by Formula (1) below or at least one second nucleotide unit represented by Formula (2) below in a 3' end region within 20 bases of the 3' end thereof and/or in a 5' end region within 25 bases of the 5' end thereof.
   (1) A base sequence represented by SEQ ID NO: 2 or a base sequence having 90% or more identity with such sequence
   (2) A base sequence consisting of between 20 and 200 consecutive nucleotides of the base sequence described in (1) above 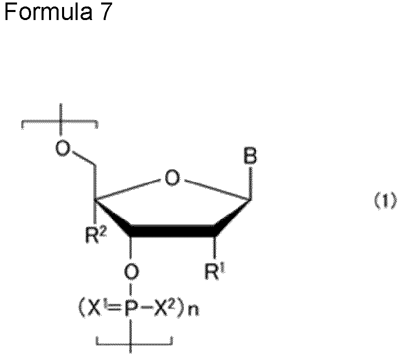 (In Formula (1), R¹ represents a hydroxyl group, a hydroxyl group with a hydrogen atom substituted with an alkyl or alkenyl group, or a halogen atom; R² represents NHR³ with a linking group, the linking group being a divalent hydrocarbon group with one or more carbon atoms; R³ represents a hydrogen atom, an alkyl group, or an alkenyl group; n is 0 or 1; X¹ represents an oxygen atom or sulfur atom; X² represents OH (or O⁻) or SH (or S⁻); and B represents a purine base or a pyrimidine base.) 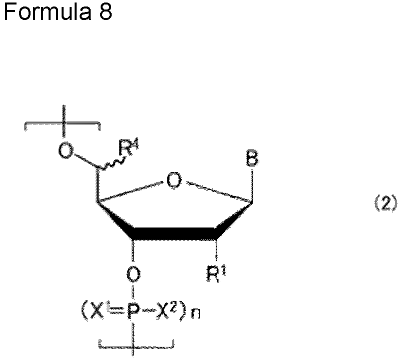 (In Formula (2), R¹ represents a hydroxyl group, a hydroxyl group with a hydrogen atom substituted with an alkyl or alkenyl group, or a halogen atom; R⁴ represents NHR⁵ with a linking group, the linking group being a divalent hydrocarbon group with one or more carbon atoms; R⁵ represents a hydrogen atom, an alkyl group, or an alkenyl group; n is 0 or 1; X¹ represents an oxygen atom or sulfur atom; X² represents OH (or O⁻) or SH (or S⁻); and B represents a purine base or a pyrimidine base.)

### EFFECTS OF THE INVENTION

Because the antitumor agent disclosed in the present specification contains, as an active ingredient, RNA having nucleotide units containing specific chemical modifications in the 3' region and/or 5' end region of the RNA, it has excellent in vivo stability. The RNA disclosed in the present specification can also efficiently interact with immune cells and activate the immune cells. This can effectively suppress tumors. The same action in such RNA can effectively activate immune cells, effectively inhibit tumor cell metastasis, effectively activate immune cells, and enhance the antitumor effect of, for example, a concomitant antitumor agent. The RNA disclosed in the present specification is useful for the prevention and/or treatment of tumors.

### BRIEF DESCRIPTION OF DRAWINGS

Figure 1 shows an overview of the synthetic RNA used in an example.
Figure 2 shows the migrating cell count when the synthetic RNA was administered to ZC3H12D-overexpressing 786-O cells.
Figure 3 shows the results of the evaluation of the effect of chemically modified RNAs on IFNγ production in human CD56⁺CD3⁻NK cells.
Figure 4 shows the results of the evaluation of the effect of chemically modified RNAs on IFNγ production in human CD56⁺CD3⁻NK cells.
Figure 5 shows an overview of the synthetic RNA used in an example.
Figure 6 shows the results of the evaluation of the RNase resistance of the chemically modified RNA in the serum of tumor-bearing mice.
Figure 7 shows the results of the evaluation of the RNase resistance of the chemically modified RNA in bovine serum.

### MODE FOR CARRYING OUT THE INVENTION

The present disclosure relates to synthetic RNA associated with exILβ-mRNA, a sequence near an untranslated region on the RNA 3' end region side of IL-1β, and uses thereof. The present inventors found that when specific nucleotide units are introduced as chemical modifications to the 3' and 5' ends of synthetic RNA corresponding to exILβ-mRNA, the NK cell activating effect is comparable to that of exIL1β-mRNA. Such synthetic RNA may play a role in vivo as a unique intercellular neurotransmitter with antitumor or anti-metastasis functions.

In the present specification, "chemical modification" of RNA can refer to any chemical modification made to RNA, and can include, for example, modifications at the ribose and phosphodiester linkages and bases of nucleotides. Bases include not only the substitution of a base sequence different from a natural base sequence, but also the chemical modification of such bases even when the type of base is the same as the base in the natural base sequence.

Representative and non-limiting examples of the present invention will now be described in detail with reference to the appropriate drawing. This detailed description is simply intended to show those skilled in the art details for embodying preferred examples of the present invention and is not intended to limit the scope of the invention. Additional features and aspects of the invention disclosed below can also be used separately from or in conjunction with other features and aspects of the invention to provide further improved antitumor agents and uses thereof.

The combinations of features and steps disclosed in the following detailed description are not essential to embodying the invention in the broadest sense, and are described below simply to illustrate representative examples of the invention in detail. Furthermore, the various features of the representative examples below, as well as the various features set forth in the independent and dependent claims, provide additional and useful embodiments of the invention. They do not have to be combined as in the specific examples listed herein or in the order in which they are listed.

All features described in the present specification and/or in the claims are intended to be disclosed separately and independently of each other as limitations to the original disclosure and specified matters in the claims, apart from the configurations of features described in the examples and/or claims. Furthermore, all references to numerical ranges, groups, or groupings are made with the intent to disclose intermediate configurations thereof as limitations to the original disclosure and specified matters in the claims.

The meaning of "lower" for the substituents in the compounds described in the present specification is a number of carbon atoms constituting the substituent up to 10. This is usually from 1 to 6 carbon atoms or 1 to 5 carbon atoms, but may also be, for example, from 1 to 4 carbon atoms or from 1 and 3 carbon atoms.

The various embodiments disclosed in the present specification will now be described in detail. The RNA used in the present specification will be described first, followed by descriptions of embodiments using the RNA.

### RNA

The RNA in the present disclosure has a base sequence selected from the group consisting of (1) and (2) below, and has at least one first nucleotide unit represented by Formula (1) below or at least one second nucleotide unit represented by Formula (2) below in a 3' end region within 20 bases of the 3' end thereof and/or in a 5' end region within 25 bases of the 5' end thereof.

The RNA in the present disclosure has the following sequences.
(1) A base sequence represented by SEQ ID NO: 2 or a base sequence having 90% or more identity with such sequence
(2) A base sequence consisting of between 20 and 200 consecutive nucleotides of the base sequence described in (1) above

The RNA consisting of the base sequence represented by SEQ ID NO: 2 corresponds to the 3' untranslated region (3'UTR) of IL1β-mRNA (full-length base sequence represented by SEQ ID NO: 1) (exIL1β-mRNA), which is the mRNA of human interleukin 1β (IL1β, NM_000576).

The RNA of the present disclosure may have the full-length base sequence represented by SEQ ID NO: 2 or may have a nucleotide sequence having 90%, 95%, 96%, 97%, 98%, or 99% or more identity to the sequence. Here, the sequence identity can be determined, for example, by alignment under optimized conditions with a highly similar sequence (megablast) using two or more sequences by blastn in the BLAST from the NCBI (BLAST: Basic Local Alignment Search Tool (nih.gov); https://blast.ncbi.nlm.nih.gov/Blast.cg).

A base sequence that has 90% or more identity with the base sequence represented by SEQ ID NO: 2 has the ability to activate NK cells from the standpoint of the present disclosure. The ability of a base sequence to activate immune cells such as NK cells can be confirmed using the method disclosed in the examples below.

The RNA of the present disclosure may be part of the base sequence represented by SEQ ID NO: 2 or a base sequence having 90% or more identity with the base sequence. (These sequences are also referred to below collectively as the sequence numbers, etc. represented by SEQ ID NO: 2.) The base sequence represented by SEQ ID NO: 2 is 605 bases in total length. A part of the base sequence represented by SEQ ID NO: 2 may be, for example, 20 and 200 consecutive base lengths from the base sequence represented by SEQ ID NO: 2. The base length may be, for example, 25 bases or more, 30 bases or more, 40 bases or more, or 45 bases or more, and, for example, 180 bases or less, 160 bases or less, 150 bases or less, 140 bases or less, 130 bases or less, 120 bases or less, 110 bases or less, 100 bases or less, 90 bases or less, 80 bases or less, 75 bases or less, 70 bases or less, or 60 bases or less. The RNA in the present disclosure can be set as a range combining one of these lower limits and upper limits as appropriate. For example, the base length can be between 20 and 100 bases long, between 30 and 100 bases long, between 30 and 80 bases long, or between 40 and 60 bases long, etc.

The range for a part of a base sequence represented by SEQ ID NO: 2 is not particularly limited, and a region with NK cell activation ability can be selected as appropriate. In the base sequence represented by SEQ ID NO: 2, for example, it can be selected from a range of 200 to 500 bases from the 5' end, 220 to 480 bases from the 5' end, 260 to 460 bases from the 5' end, and 300 to 450 bases from the 5' end, etc.

Such base sequences include, for example, those represented by SEQ ID NO: 4, SEQ ID NO: 5, and SEQ ID NO: 6, as well as base sequences that have 90% or more, 95% or more, 96% or more, 97% or more, 98% or more, or 99% or more identity to these base sequences. From the standpoint of the present disclosure, parts of the base sequence represented by SEQ ID NO: 2 have the ability to activate NK cells. The mRNA consisting of the sequence represented by SEQ ID NO: 5 is one of the fragments of human IL1 β-mRNA obtained when divided into four fragments containing 150 nucleotides. This also includes the base sequence represented by SEQ ID NO: 10 and base sequences that have 90% or more, 95% or more, 96% or more, 97% or more, 98% or more, and 99% or more identity with this base sequence.

### Chemical Modification of RNA

### First Nucleotide Unit: Nucleotide Unit Represented by Formula (1)

In the nucleotide unit represented by Formula (1), R¹ represents a hydroxyl group, a hydroxyl group in which a hydrogen atom is substituted with an alkyl group or alkenyl group, or a halogen atom.

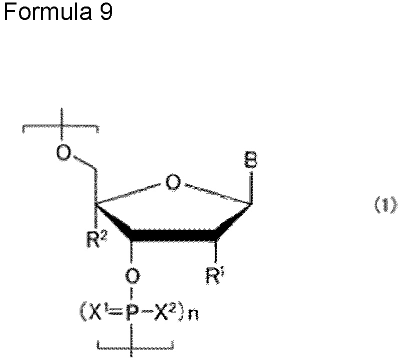

The alkyl groups include saturated hydrocarbon groups that are linear, branched, cyclic, or a combination thereof. Usually, lower alkyl groups are preferred. Lower alkyl groups with 1 to 6 carbon atoms and lower alkyl groups with 1 to 5 carbon atoms are preferred, and lower alkyl groups with 1 to 4 carbon atoms and lower alkyl groups with 1 to 3 carbon atoms are especially preferred. Linear alkyl groups with 1 to 4 carbon atoms include methyl, ethyl, n-propyl, and n-butyl groups. Among these, methyl, ethyl, and n-propyl groups are preferred, methyl and ethyl groups are more preferred, and a methyl group is especially preferred. Branched alkyl groups with 1 to 4 carbon atoms include isopropyl, isobutyl, s-butyl, and t-butyl groups. Among these, an isopropyl groups is especially preferred. The cyclic alkyl groups with 1 to 4 carbon atoms include cyclopropyl, cyclobutyl, and cyclopropylmethyl groups.

The alkenyl groups include saturated hydrocarbon groups that are linear, branched, cyclic, or a combination thereof. Usually, lower alkenyl groups are preferred. Lower alkenyl groups include, for example, ethenyl, 1-propenyl, 2-propenyl, 1-methyl-2-propenyl, 1-methyl-1-propenyl, 2-methyl-1-propenyl, 1-butenyl, and 2-butenyl groups.

Halogen atoms include, but are not limited to, fluorine atoms.

R² can be NHR³ with a linking group. In other words, NHR³ is attached to the carbon atom at position 4' via a linking group attached to the nitrogen atom.

Linking groups include divalent hydrocarbon groups with one or more carbon atoms. Divalent hydrocarbon groups include alkylene groups with 1 to 8 carbon atoms and alkenylene groups with 2 to 8 carbon atoms.

An alkylene group serving as a linking group may be linear or branched, but is preferably linear. Lower alkylene groups with 1 to 6 carbon atoms are preferred. Lower alkylene groups with 1 to 6 carbon atoms are preferred, lower alkylene groups with 2 to 6 carbon atoms are more preferred, and lower alkylene groups with 2 to 4 carbon atoms or 2 to 3 carbon atoms are especially preferred. Linear alkylene groups 1 to 4 carbon atoms include methylene, ethylene, propane-1,3-diyl, n-butane-1,1-diyl, n-pentyl-1,5-diyl, and n-hexyl-1,6-diyl groups. Another example is a butane-1,2-diyl group. Ethylene, propane-1,3-diyl, and n-butane-1,1-diyl groups are especially preferred.

An alkenylene group serving as a linking group can be linear or branched, but is preferably linear. Lower alkenylene groups are preferred. Lower alkenylene groups include ethene-1,2-diyl, propene-1,3-diyl, and butene-1,4-diyl groups.

In the nucleotide unit represented by Formula (1), a divalent hydrocarbon group, such as an alkylene group having from 1 to 3 carbon atoms, is preferred from the standpoint of the NK cell activation ability of the RNA in the present disclosure. Examples include methylene, ethylene, and propylene groups.

R³ can be a hydrogen atom, alkyl group, or alkenyl group. In addition to the alkyl groups already described, lower alkyl groups are preferred, such as alkyl groups with 1 to 3 carbon atoms, alkyl groups with 1 or 2 carbon atoms, and methyl groups. In addition to the alkenyl groups already described, lower alkenyl groups are preferred as alkenyl groups. When R³ is a hydrogen atom or one of these groups, the linking group is preferably an alkylene group with 2 or more carbon atoms, 3 or more carbon atoms, or 4 or more carbon atoms, and 6 or fewer carbon atoms, 5 or fewer carbon atoms, or 4 or fewer carbon atoms.

When R³ is a hydrogen atom, R² is NH2 (an amino group) with a linking group, that is, an aminoalkyl group when the linking group is an alkylene group. In Formula (1), R² as an aminoalkyl group, such as an aminoethyl group or an aminopropyl group, is considered ideal for the RNA of the present disclosure.

Here, n represents 0 or 1. When n is 0, the 3' position of the nucleotide unit may be O⁻, for example, OH. When n is 1, X¹ represents an oxygen or sulfur atom and X² represents OH (or O⁻) or SH (or S⁻). When X¹ or X² is a sulfur atom or SH(S⁻), the nucleotide unit will have the next nucleotide unit with a phosphorothioate bond on the 3' side. When n is 1, the nucleotide unit can have a phosphate or phosphorothioate group at the 3' position. When it has a phosphate group, it may be a salt with an appropriate base.

B represents a purine base or pyrimidine base. Purine bases include, but are not particularly limited, to purin-9-yl groups and various substituted purin-9-yl groups such as 2,6-dimethoxypurin-9-yl and 2,6-dichloropurin-9-yl. The pyrimidine bases include 2-oxo-pyrimidin-1-yl and 2-oxo-pyrimidin-1-yl groups. This also includes substituted 2-oxo-pyrimidin-1-yl groups such as 2-oxo-4-methoxy-pyrimidin-1-yl and 4-(1H-1,2,4-triazol-1-yl)-pyrimidin-1-yl.

B may also be a modified base as described below.

### Second Nucleotide Unit: Nucleotide Unit Represented by Formula (2)

The second nucleotide unit represented by Formula (2) below is more specifically a nucleotide unit represented by either of Formulas (2a) and 2(b) below. The nucleotide unit represented by Formula (2a) is an R-body with respect to the chiral carbon atom at position 5', and the nucleotide unit represented by Formula (2b) is an S-body with respect to the chiral carbon atom at position 5'. In the RNA disclosed in the present specification, these nucleotide units may be used with either an R-body or an S-body, or a combination of these. For example, it may be preferrable for the RNA disclosed in the present invention to use only an S-body as the nucleotide unit represented by Formula (2) from the standpoint of nuclease resistance, stability in serum, and thermal stability of the RNA/RNA duplex. In the present specification, the nucleotide unit represented by Formula (2) and the second nucleotide unit refers to an S-body and/or R-body, unless an S-body or R-body is explicitly mentioned.

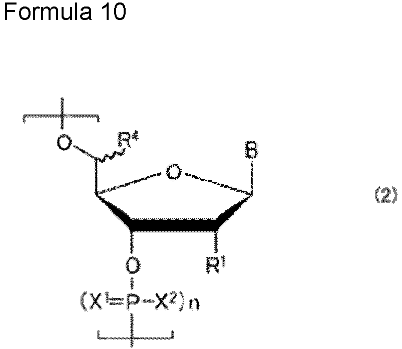

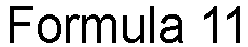

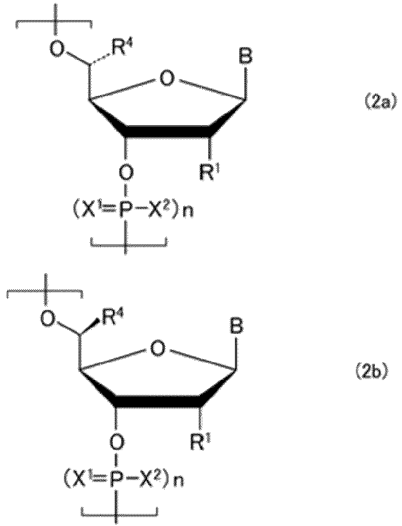

In the second nucleotide unit, R¹ has the same meaning as in Formula (1). R¹ may preferably represent a hydroxyl group in which a hydrogen atom has been substituted by an alkyl group or an alkenyl group, or a halogen atom. Among these, R¹ may preferably be a hydroxyl group in which the hydrogen atom has been substituted with an alkyl group containing from 1 to 3 carbon atoms.
For example, methyl, ethyl, and n-propyl groups are preferred, methyl and ethyl groups are more preferred, and methyl groups are especially preferred. There are no particular limitations on the halogen atom, but a fluorine atom may be preferred.

In the second nucleotide unit, R⁴ can represent NHR⁵ with a linking group. In other words, NHR⁵ is bound to the carbon atom at the 5' position via a linking group that is bound to the nitrogen atom.

In the second nucleotide unit, the linking group and R⁵ are equivalent to the linking group and R³ regarding R² in Formula (1). The linking group may be, for example, a lower alkylene group, a lower alkylene group with 1 to 6 carbon atoms, a lower alkylene group with 2 to 6 carbon atoms, or a lower alkylene group with 2 to 4 carbon atoms or 2 to 3 carbon atoms. Examples include methylene, ethylene, propane-1,3-diyl, n-butane-1,1-diyl, n-pentyl-1,5-diyl, and n-hexyl-1,6-diyl groups. Another example is a butane-1,2-diyl group. An ethylene, propane-1,3-diyl, or n-butane-1,1-diyl groups may be preferred, and a propane-1,3-diyl group may be especially preferred.

An alkenylene group serving as the linking group may be linear or branched, but is preferably linear. A lower alkenylene group may be preferred, and examples of lower alkenylene groups include ethene-1,2-diyl, propen-1,3-diyl, and butene-1,4-diyl groups.

In the second nucleotide unit, a methylene group, ethylene group, or a divalent hydrocarbon group such as an alkylene group with 1 to 3 carbon atoms (for example, a propane-1,3-diyl group) is ideal from the standpoint of the NK cell activation ability of the RNA in the present disclosure.

In the second nucleotide unit, R⁵ may preferably be a hydrogen atom, an alkyl group with 1 to 3 carbon atoms, an alkyl group with 1 or 2 carbon atoms, or a methyl group. When R⁵ is a hydrogen atom or one of these groups, the linking group may preferably be an alkylene group with 2 carbon atoms or more, 3 carbon atoms or more, or 4 carbon atoms or more, and 6 carbon atoms or less, 5 carbon atoms or less, or 4 carbon atoms or less. Ethylene and propane-1,3-diyl groups may be preferred.

Also, when R⁵ is a hydrogen atom; R⁴ is NH₂ (an amino group) with a linking group, that is, an aminoalkyl group when the linking group is an alkylene group. In Formula (2), R⁴ is an aminoalkyl group such as an aminoethyl group or an aminopropyl group, which is ideal for the RNA in the present disclosure.

In the second nucleotide unit, X¹ and X² have the same meaning as those in the first nucleotide unit, and a similar arrangement can be adopted. In the second nucleotide unit, n represents 0 or 1. The second nucleotide unit can have the same arrangement as the first nucleotide unit when n is 0 or 1. In addition, B has the same meaning as B in the first nucleotide unit, and a similar arrangement can be adopted.

In the present disclosure, the RNA has at least one nucleotide unit represented by Formula (1) below or at least one nucleotide unit represented by Formula (2) below in a 3' end region within 25 bases of the 3' end thereof and/or in a 5' end region within 25 bases of the 5' end thereof. The RNA in the present disclosure may have either of these nucleotide units only in the 5' end region, either of these nucleotide units only in the 3' end region, or either of these nucleotide units only in both the 5' and 3' end regions.

In addition, the RNA in the present disclosure may have a combination of a first and second nucleotide units in the 5' end region. It may also have a combination of first and second nucleotide units in the 3' end region. The RNA in the present disclosure may also have a combination of first and second nucleotide units in both the 3' and 5' end regions as appropriate. For example, the 5' and 3' end regions may each have a first and second nucleotide units, or vice versa.

In the 5' end region, it may be, for example, within 20 bases, within 15 bases, or within 10 bases of the 5' end. It may also be, for example, within 8 bases, within 6 bases, or within 4 bases. It may also be, for example, within 3 bases, within 2 bases, or within 1 base (at the 5' end). In the 3' end region, it may be, for example, within 20 bases or within 15 bases or within 10 bases of the 3' end. It may also be, for example, within 10 bases, within 8 bases, or within 6 bases. It may also be, for example, within 5 bases or within 4 bases. It may also be, for example, within 3 bases, within 2 bases, or within 1 bases (at the 3' end).

The RNA in the present disclosure has at least one nucleotide unit represented by Formula (1) and/or nucleotide unit represented by Formula (2) in the 5' end region. The number of nucleotide units represented by the nucleotide units represented by Formula (1) and Formula (2) in this region is not particularly limited, but there may be from 1 to 6, from 1 to 5, from 1 to 4, from 1 or 3, or 1 or 2. The nucleotide units represented by Formula (1) and Formula (2) are provided, for example, in a region within up to 6 bases of the 5' end. In this way, it is believed that the 5' end can be effectively protected. The nucleotide units represented by Formula (1) and Formula (2) may be effective if, for example, they are provided at least at the 5' end or two bases from the 5' end.

The RNA in the present disclosure has at least one nucleotide unit represented by Formula (1) and/or nucleotide unit represented by Formula (2) in the 3' end region. The number of nucleotide units represented by Formula (1) and Formula (2) in this region is not particularly limited, but there may be from 1 to 6, from 1 to 5, from 1 to 4, from 1 or 3, or 1 or 2. The nucleotide units represented by Formula (1) and Formula (2) are provided, for example, in a region within up to 6 bases of the 3' end. In this way, it is believed that the 3' end can be effectively protected. The nucleotide units represented by Formula (1) and Formula (2) may be effective if, for example, they are provided at least at the 3' end or two bases from the 5' end.

The RNA in the present disclosure can have at least one nucleotide unit represented by Formula (1) and/or one nucleotide unit represented by Formula (2) in both the 3' end region and the 5' end region. Because the RNA has these nucleotide units in their end regions, it is highly resistant to nucleases, and as a result, exhibits excellent antitumor and metastasis suppressing effects. The number of nucleotide units represented by Formula (1) and Formula (2) that are provided in each of these end regions can be, for example, 1 to 5, 1 to 4, 1 to 3, or 1 to 2. In each of these end regions, one or two nucleotide units represented by Formula (1) and/or one or two nucleotide units represented by Formula (2) within two or three bases of each end may be preferred. It may also be preferable for at least one or all of the nucleotide units represented by Formula (1) provided in each of these end regions to have a fluorine atom at the 2' position of the ribose and an aminoethyl group at the 4' position. It may also be preferable for at least one or all of the nucleotide units represented by Formula (2) provided in each end region to have a methoxy group at the 2' position of the ribose and an aminopropyl group at the 5' position. It may also be preferable to have a nucleotide unit represented by Formula (2) with an amino propyl group at the 5' position in the 5' end region of RNA one or two places from the 5' end, and to have a nucleotide unit represented by Formula (1) with an amino ethyl group at the 4' position in the 3' end region of RNA one or two places from the 3' end. This arrangement is preferable because the aminoalkyl groups are closer to the 5' and 3' ends, which are exposed to nucleases. In addition, the types of bases in these nucleotide units is not particularly limited, but it may be preferable to use a base with a pyrimidine ring, or may be preferable to use cytosine or uracil.

Based on the above, the following arrangements may be suitable for use in the RNA of the present disclosure.
[1] The nucleotide unit represented by Formula (1) is a nucleotide unit in which n is 1, R¹ is a fluorine atom, and R² is an aminoethyl group;
   the nucleotide unit represented by Formula (2) is a nucleotide unit in which R¹ is a methoxy group and R⁴ is an amino propyl group;
   and at least one of the nucleotide unit represented by Formula (1) and/or the nucleotide unit represented by Formula (2) is present within two bases of the 3' end of the 3' end region, and at least one is present within two bases of the 5' end of the 5' end region;
   in particular, having a nucleotide unit represented by Formula (2) in the 5' end region and a nucleotide unit represented by Formula (1) in the 3' end region
[2] In [1], the base sequence has at least 90% identity with a base sequence of 30 to 70 consecutive bases within the base sequence represented by SEQ ID NO: 4
[3] In [1], the base sequence has at least 90% identity with a base sequence of 40 to 60 consecutive bases within the base sequence represented by SEQ ID NO: 4
[4] In [1] to [3], the base sequence is a base sequence that has at least 90% identity with the base sequence represented by SEQ ID NO: 6 or SEQ ID NO: 10
[5] In [1] to [4], a base in the nucleotide units is either cytosine or uracil

In the 5' and 3' end regions of the RNA in the present disclosure, in addition to having a nucleotide unit represented by Formula (1) and/or a nucleotide unit represented by Formula (2), the ribose can have a structure linked by a phosphorothioate bond. The linkage by a phosphorothioate bond can be present in both the nucleotide unit represented by Formula (1) and the nucleotide unit represented by Formula (2), but it may be advantageous for nucleotides corresponding to other bases that are not substituted by the nucleotide unit to also be linked by a phosphorothioate bond.

The number and location of the phosphorothioate bonds introduced in the 5' and 3' end regions are not particularly limited. For example, there may be 1 to 8, 1 to 6, 1 to 5, 1 to 4, or 1 to 3 consecutively, including the initial linked portion from the 5' end and 3' end, etc. In addition to the first linked portion from the end, there may also be 1 to 4 in one or two bases.

The RNA in the present disclosure may have a 5' end region and/or a 3' end region in which, in addition to having at least one nucleotide unit represented by Formula (1) and/or a nucleotide unit represented by Formula (2), the other nucleotide unit has a natural nucleotide, that is, a nucleotide unit that has natural ribose. This may be, for example, a nucleotide unit in which the OH group in the ribose has been modified, such as the introduction of a methoxy group at the 2' position of the ribose, or a nucleotide unit in which another known chemical modification has been made to the ribose. For example, the RNA in the present disclosure may be RNA that has chemically modified ribose having a methoxy group at the 2' position of the ribose in all nucleotide units other than the nucleotide unit represented by Formula (1) provided in the 5' and/or 3' end regions.

From the above, for the RNA in the present disclosure, it may be preferable, for example, that all nucleotide units other than the nucleotide units specified above have a chemically modified ribose with a methoxy group at the 2' position of the ribose in any of arrangements (1) to (5) above.

The RNA in the present disclosure can also be provided with chemical modifications that make the uridine in the RNA a uridine analog. The number of chemically modified uridine analogues replacing uridine is not limited to a percentage of the total number of uridines in the RNA of the present disclosure. For example, 80% or more, 90%, or more or 100% of the total number of uridines can be uridine analogues.

Nucleobases and nucleosides containing uridine analogues include, but are not limited to, uridine analogues selected from the group consisting of uridine pseudouridine, 1-methylpseudouridine, 1-ethylpseudouridine, 2-thiouridine, 4'-thiouridine, 5-methylcytosine, 2-thio-1-methyl-1-deaza-pseudouridine, 2-thio-1-methyl-pseudouridine, 2-thio-5-aza-uridine, 2-thio-dihydro-pseudouridine, 2-thio-dihydro-uridine, 2-thio-pseudouridine, 4-methoxy-2-thio-pseudouridine, 4-methoxy-pseudouridine, 4-thio-1-methyl-pseudouridine, 4-thio-pseudouridine, 5-aza-uridine, dihydro-pseudouridine, 5-methoxy-uridine, and 2'-O-methyluridine. For example, the uridine analog may be 1-methyl-pseudouridine.

The RNA of the present disclosure can be further provided with chemical modifications that make cytidine in the RNA into a cytidine analog. Chemical modifications that make cytidine into cytidine analogues are not limited to a percentage of the total number of cytidines in the RNA of the present disclosure. For example, 80% or more, 90% or more, or 100% of the total number of cytidines can be made into cytidine analogues.

Nucleobases and nucleosides containing cytidine analogues include 5-aza-cytidine, 6-aza-cytidine, pseudoisocytidine, 3-methyl-cytidine, 4-acetyl-cytidine, 5-formyl-cytidine, 4-methyl-cytidine, 5-methyl-cytidine, 5-halo-cytidine, 5-hydroxymethyl-cytidine, 1-methyl-pseudoisocytidine, pyrrolo-cytidine, pyrrolo-pseudoisocytidine, 2-thio-cytidine, 2-thio-5-methyl-cytidine, 4-thio-pseudoisocytidine, 4-thio-1-methyl-pseudoisocytidine, 4-thio-1-methyl-1-deaza-pseudoisocytidine, 1-methyl-1-deaza-pseudoisocytidine, zebularine, 5-aza- zebularine, 5-methyl- zebularine, 5-aza-2-thio-zebularine, 2-thio- zebularine, 2-methoxy-cytidine, 2-methoxy-5-methyl-cytidine, 4-methoxy-pseudoisocytidine, 4-methoxy-1-methyl-pseudoisocytidine, lysidine, α-thio-cytidine, 2'-O-methyl-cytidine, 5,2'-O-dimethyl-cytidine, 4-acetyl-2'-O-methyl-cytidine, 4,2'-O-dimethyl-cytidine, 5-formyl-2'-O-methyl-cytidine (f5Cm), 4,4,2'-O-trimethyl-cytidine, 1-thio-cytidine, 2'-F-aracytidine, 2'-F-cytidine, and 2'-OH-aracytidine.

The RNA of the present disclosure can further be provided with chemical modifications that make the adenine in the RNA into an adenine analog. Chemical modifications that make adenine into adenine analogues are not limited to a percentage of the total number of adenine in the RNA of the present disclosure. For example, 80% or more, 90% or more, or 100% of the total number of adenine can be made into cytidine analogues.

Nucleobases and nucleosides containing adenine analogues include 2-aminopurine, 2,6-diaminopurine, 2-amino-6-halopurine (for example, 2-amino-6-chloropurine), 6-halopurine (for example, 6-chloropurine), 2-amino-6-methyl-purine, 8-azido-adenosine, 7-deaza-adenine, 7-deaza-8-aza -adenine, 7-deaza-2-amino-purine, 7-deaza-8-aza-2-amino-purine, 7-deaza-2,6-diaminopurine, 7-deaza-8-aza-2,6-diaminopurine, 1-methyladenosine, 2-methyl-adenine, 6-methyladenosine, 2-methylthio-6-methyladenosine, 6-isopentenyladenosine, 2-methylthio-6-isopentenyl-adenosine, 6-(cis-hydroxyisopentenyl)adenosine, 2-methylthio-6-(cis-hydroxyisopentenyl) adenosine, 6-glycinylcarbamoyl adenosine, N6-threonylcarbamoyl adenosine, 6-methyl-N6-threonylcarbamoyl-adenosine, 2-methylthio-6-threonylcarbamoyl-adenosine, 6,6-dimethyl-adenosine, 6-hydroxy-norvalylcarbamoyl-adenosine, 2-methylthio-6-hydroxy-norvalylcarbamoyl-adenosine, 6-acetyl-adenosine, 7-methyladenine, 2-methylthio-adenine, 2-methoxy-adenine, alpha-thio-adenosine, 2'-O-methyl-adenosine, 6,2'-O-dimethyl-adenosine, 6,6,2'-O-trimethyl-adenosine, 1,2'-O-dimethyl-adenosine, 2'-O-ribosyl-adenosine (phosphate), 2-amino-N6-methyl-purine, 1-thio-adenosine, 8-azido-adenosine, 2'-F-ara-adenosine, 2'-F-adenosine, 2'-OH-ara-adenosine, and 6-(19-amino-pentaoxanadecyl)-adenosine.

The RNA of the present disclosure can further be provided with chemical modifications that make the guanine in the RNA into a guanine analog. Chemical modifications that make guanine into guanine analogues are not limited to a percentage of the total number of guanine in the RNA of the present disclosure. For example, 80% or more, 90% or more, or 100% of the total number of guanine can be made into guanine analogues.

Nucleobases and nucleosides containing guanine analogues include inosine, 1-methyl-inosine, wyosine, methyl wyosine, 4-demethyl-wyosine, isowyosine, wybutosine, peroxy wybutosine, hydroxy wybutosine, unmodified hydroxy wybutosine, 7-deaza-guanosine, queuosine, epoxyqueuosine, galactosyl-queuosine, mannosyl-queuosine, 7-cyano-7-deaza-guanosine, 7-aminomethyl-7-deaza-guanosine, archaeosine, 7-deaza-8-aza-guanosine, 6-thio-guanosine, 6-thio-7-deaza-guanosine, 6-thio-7-deaza-8-azaguanosine, 7-methylguanosine, 6-thio-7-methyl-guanosine, 7-methyl-inosine, 6-methoxy-guanosine, 1-methylguanosine, 2-methylguanosine, 2,2-dimethylguanosine, 2,7-dimethylguanosine, 2,2,7- dimethyl-guanosine, 8-oxo-guanosine, 7-methyl-8-oxo-guanosine, 1-methyl-6-thio-guanosine, 2-methyl-6-thio-guanosine, 2,N2-dimethyl-6-thio-guanosine, alpha-thio-guanosine, 2'-O-methyl-guanosine, 2-methyl-2'-O-methyl-guanosine, 2,2-dimethyl-2'-O-methyl-guanosine, 1-methyl-2'-O-methyl-guanosine, 2,7-dimethyl-2'-O-methyl-guanosine, 2'-O-methyl-inosine, 1,2'-O-dimethyl-inosine, 2'-O-ribosylguanosine (phosphate), 1-thio-guanosine, O6-methyl-guanosine, 2'-F-ara-guanosine, and 2'-F-guanosine.

The RNA in the present disclosure may have a 5' untranslated region (UTR) at the 5' end of a first region, when the base sequence defined in (1) and (2) is established as the first region. The 5' UTR may be the natural 5' UTR of the polypeptide encoded by the first region, or may be a 5' UTR different from that of the encoded polypeptide. For example, the 5' UTR may contain at least one translation initiation sequence, such as a Kozak sequence, an internal ribosomal entry site (IRES), and/or fragments thereof. Sequences and designs for these 5' UTRs in RNA medicines are commonly known.

The RNA in the present disclosure may also include a 5' cap and a polyA tail. A 5' cap is a common component in modified nucleotides and generally refers to a structure that is added to the 5' end of mature mRNA.

The 5' cap can be formed by a modified nucleotide, especially a derivative of a guanine nucleotide. The 5' cap is preferably bound to the 5' end via a 5'-5'-triphosphate bond. The 5' cap can be methylated, for example, m7GpppN, where N is the 5' nucleotide at the end of a nucleic acid with a 5' cap.

A polyA tail is also known as a polyA tail portion, and refers to a series of adenine polynucleotides. The length of the polyA base is not particularly limited, but 20 or more is preferable, and 50 or more is even more preferable. The upper limit of the base length is not particularly limited, and can be, for example, 200 or less.

The RNA in the present disclosure may be mRNA or a portion thereof. When mRNA or a portion thereof, a 5' UTR and/or polyA tail may be included. When mRNA, the RNA in the present disclosure is a variant of RNA having the base sequence represented by SEQ ID NO: 1 in the coding region, wherein the coding sequence from the 88th to 897th base in the base sequence represented by SEQ ID NO: 1 has bases substituted to have 1 to 5 additional stop codons compared to the natural coding sequence. An example is a base sequence represented by SEQ ID NO: 3 (containing three stop codons). The RNA in the present disclosure may include a G→A mutation to avoid a sequence of bases such as G, if necessary.

The RNA in the present disclosure may be an RNAi inducer, RNAi agent, siRNA, shRNA, miRNA, antisense RNA, RNA, aptamer, or vector.

The RNA in the present disclosure may have labeling molecules such as fluorescent substances or chromogenic substances attached to portions thereof.

The chemical modifications described above are well-known or commonly known to those skilled in the art, and a person skilled in the art can obtain RNA with the desired chemical modification based on what is disclosed in the present specification and common technical knowledge at the time of filing of the present application. RNA molecules provided with a nucleotide unit represented by Formula (1) and/or a nucleotide unit represented by Formula (2) can be produced using the methods described in WO 2018/110678 A1 and WO 2019/088179 A1.

When the RNA in the present disclosure is administered to cells overexpressing the ZC3H12D protein, it can activate cell migration, induce anti-metastatic activity in cells, and increase tumor-destroying activity in the case of natural killer (NK) cells.

The various embodiments of the RNA in the present disclosure can be applied to each of the following embodiments of the RNA in the present disclosure, either alone or in combination.

### Antitumor Agent and Tumor Growth Inhibiting Method

The antitumor agent of the present disclosure contains the RNA of the present disclosure as an active ingredient. The method of inhibiting the growth of tumor cells in the present disclosure is to administer the RNA of the present disclosure to humans or non-human animals in vitro. The RNA in the present disclosure has the ability to activate immune cells when administered to humans or non-human animals in vitro, and therefore has an antitumor effect.

The RNA in the present disclosure as an active ingredient of an antitumor agent is RNA that can activate immune cells or increase the antitumor activity of cells. The cells can be cancer cells, tumor cells, or immune cells, and immune cells include natural killer (NK) cells, macrophages, eosinophils, neutrophils, basophils, dendritic cells, and lymphocytes. Preferably, the immune cells are natural killer cells (NK cells), macrophages, or both.

Tumors include those of head and neck cancer, esophageal cancer, gastric cancer, colon cancer, rectal cancer, liver cancer, gallbladder cancer, bile duct cancer, biliary tract cancer, pancreatic cancer, lung cancer, breast cancer, ovarian cancer, cervical cancer, uterine cancer, renal cancer, bladder cancer, prostate cancer, testicular cancer, osteosarcoma, multiple myeloma, skin cancer, brain tumors, and mesothelioma.

The activation of immune cells can be evaluated by factors such as IFNγ production by immune cells and improvement in the migration ability of immune cells or tumor cells expressing the ZC3H12D protein. Antitumor activity can be evaluated using a known method for evaluating antitumor activity.

### Immune Cell Activator (Stimulator) and Immune Cell Activating (Stimulating) Method

The immune cell activator of the present disclosure contains the RNA of the present disclosure as an active ingredient. The method of activating immune cells in the present disclosure may include administering the RNA of the present disclosure to a human or non-human animal in vitro. The previously described embodiments of antitumor agents can be applied to the activation of cells and immune cells.

### Tumor Cell Metastasis Inhibitor and Tumor Cell Metastasis Inhibiting Method

The tumor cell metastasis inhibitor of the present disclosure contains the RNA of the present disclosure as an active ingredient. The method for inhibiting tumor cell metastasis disclosed in the present specification may include administering the RNA of the present disclosure to a human or non-human animal in vitro. The RNA of the present disclosure can inhibit tumor cell metastasis by activating immune cells to increase the antitumor activity of the cells. The previously described embodiments of antitumor agents can be applied to cells, immune cells, immune cell activation, and antitumor activity.

Inhibition of tumor cell metastasis can be evaluated by measuring the number of metastatic tumor cells and other known methods.

A treatment intended to inhibit metastasis of the primary cancer with a metastasis inhibitor may be provided in combination with treatment of the primary cancer or independently. For example, when a metastasis inhibitor is used in combination, it can be used before or after or concurrently with one or more of the following: surgical treatment of the primary cancer, treatment with an anticancer drug, radiation therapy (X-rays, protons, heavy particle beams), and electromagnetic wave therapy (radio waves). Combination with surgical treatment is one of the preferred approaches.

Typically, this is one of the following.
(a) When the primary cancer remains after surgical treatment of the primary cancer, it is used in combination with one or more of a anticancer drug therapy, radiation therapy, and electromagnetic wave therapy.
(b) When the primary cancer can be removed after surgical treatment of the primary cancer, it is used alone or in combination with one or more of an anticancer drug therapy, radiation therapy and electromagnetic wave therapy to inhibit metastasis.
(c) If new primary cancer or metastasis is found after surgical treatment of the primary cancer, it is used alone or in combination with one or more of a surgical treatment, anticancer drug treatment, radiation therapy, and electromagnetic wave therapy to inhibit metastasis.

### Immunostimulatory Effect Enhancer for Immunostimulants and Effect Enhancing Method for Immunostimulants

The enhancer of the present disclosure contains the RNA of the present disclosure as an active ingredient. The methods of enhancement disclosed in the present specification may include administering the RNA of the present disclosure to a human or non-human animal in vitro. The RNA of the present disclosure is RNA that can activate immune cells, increase the antitumor activity of cells, or inhibit tumor cell metastasis. Therefore, it can be used in combination with an immune cell activator, antitumor agent, or metastasis inhibitor to enhance the immune cell activation, anti-tumor activity, or metastasis inhibitory effect of these agents, and embodiments of the antitumor agent and metastasis inhibitor described above can be applied to the activation of cells or immune cells, antitumor activity, and inhibition of metastasis.

A known immunostimulant can be the immunostimulant used in combination. A known metastasis inhibitor can be the metastasis inhibitor in combination.

The antitumor agent used in combination can be one or more of the following: a kinase inhibitor, an apoptosis inducer, a nuclear receptor modulator, an immunomodulator, a nuclear export signal inhibitor, a proteasome modulator, a DNA disrupting agent, a metabolic antagonist, a platinum-based antitumor agent (platinum complex), a microtubule inhibitor, an alkylating agent, and an anthracycline-based antitumor agent. Salts of these compounds are also included. These antitumor agents are well known and commercially available.

The method of administration of a concomitant drug with the enhancer of the present disclosure is not particularly limited as long as the prescribed enhancing effect of the enhancer of the present disclosure is realized, and they can be administered simultaneously, sequentially, or at intervals. The order of administration of these compositions is also not particularly limited, and the antitumor agent may be administered before, concurrently with, or after the RNA.

A concomitant drug and the enhancer of the present disclosure may be used together as individual drug dosages, or may be provided as a fixed-dose combination or a drug kit with a defined dosage regimen.

### Pharmaceutical Composition, Method for Improving Immunity, and Method for Preventing or Treating Tumors

The pharmaceutical composition disclosed in the present specification contains this RNA as an active ingredient. The method for improving immunity and the method for preventing or treating tumors disclosed in the present specification may include administering the RNA of the present disclosure to a human or non-human animal in vitro. The RNA in the present disclosure is RNA that can activate immune cells, increase the antitumor activity of cells, or inhibit tumor cell metastasis. Therefore, a pharmaceutical composition containing these is useful for the prevention or amelioration of various conditions and diseases by activation of immune cells, etc., as well as for the prevention or treatment of diseases such as tumors by antitumor activity and tumor metastasis inhibition activity. The tumors include those that have already been mentioned.

The previously described embodiments can be applied to the activation of cells and immune cells, antitumor activity, and inhibition of tumor cell metastasis.

In using the RNA of the present disclosure as an active ingredient in a pharmaceutical product, various dosage forms can be developed according to the prophylactic or therapeutic purpose by including a pharmacological carrier as necessary. The dosage form of an antitumor agent containing the RNA can be, for example, an injectable, suppository, oral formulation, ointment, or eye drops, preferably an injectable (intravenous injection, etc.). Each of these dosage forms can be produced using production methods commonly known to those skilled in the art.

Any organic or inorganic carrier material commonly used in pharmaceutical formulations can be used as the pharmacological carriers. Examples include an excipient, binder, disintegrant, lubricant, or coating agent, etc. in solid formulations, and a solvent, dissolution aid, suspending agent, isotonic agent, pH adjuster, pH buffer, or analgesic, etc. in liquid formulations. Formulation additives such as preservatives, antioxidants, colorants, flavor enhancers, odor enhancers, and stabilizers can also be used as needed.

The target of administration of the various agents and pharmaceutical compositions in the embodiments of the present invention is mammals, preferably humans.

The daily dosage of RNA depends on the patient's symptoms, weight, age, and gender, etc. and cannot be determined in general. However, usual daily dosage of the compound for an adult (50 kg body weight) is preferably 10 ng to 1 mg, more preferably 100 ng to 100 µg, and even more preferably 1 µg to 10 µg.

The amount of RNA to be added to each of the dosage unit forms mentioned above is set according to the nature of the RNA used, the symptoms of the patient, and the dosage form.

The disclosures of all patent applications and references documents cited in the present specification are incorporated herein by reference in their entirety.

The present disclosure will now be described in greater detail with reference to examples. The present disclosure is not limited to these examples.

### EMBODIMENTS

Unless otherwise noted, the following methods were followed.

### (1) Reagent

The primary antibody and factor used in the present research: human anti-ZC3H12D antibody (ab1000862; Abcam); cell sorting for human CD56: PE anti-human CD56 (NCAM) antibody (BioLegend) and isotype control, mouse IgG1, κ (BioLegend); cell sorting for human CD3: AlexaFluor 488 anti-human CD3 antibody (BioLegend) and isotype control, mouse IgG1, κ (BioLegend). Mouse (D-17, sc-9344; Santa Cruz Biotechnology) and human (B27; BioLegend) anti-IFNγ antibodies were used for cell staining. Human IL2 (BioLegend) and human IL12 (PeproTech) were used for cell culture.

### (2) Tumor Cell Line and Tumor Cell Culture Supernatant

Human renal carcinoma 786-O cells were purchased from ATCC. The human cells were cultured in DMEM/Han's F-12 medium.

### (3) Primary Human Cell Culture

Primary human BMCs (HPBMCs; Lonza) were cultured in LGM-3 medium (Lonza) supplemented with 200 IU/mL of IL2.

### (4) Migration Assay

Cell migration was assessed using a chemotaxis Boyden chamber (NeuroProbe). The upper and lower wells were separated by 5-µm pore size polyvinylpyrrolidone-free polycarbonate filters (Nucleopore, Costar). Various types of RNA were applied to the lower wells. An aliquot (50 µL) of cell suspension (from 2 × 10⁵ to 2 × 10⁶ cells/mL) was inoculated into each of the upper wells and incubated for 3.5 hours.

### (5) IFNγ Induction Assay

After stimulation with 50 ng/mL RNA in LGM-3 medium (Lonza) supplemented with 200 IU/mL IL2 for 48 hours, human CD56⁺CD3⁻NK cells were stained with anti-human IFNγ antibody. Cells were incubated with 40 ng/mL hIL-12 for 48 hours for a IFNγ-induced positive control.

### (6) Vector Construction and Establishment of Cells Stably Expressing ZC3H12D

Human ZC3H12D expression vectors (hZC58 and hZC36) were cloned in a human ZC3H12D coding region pCMV6-entry vector (C-terminalmyc-FLAGtag), which was purchased from OriGene Technologies Inc. (Rockville, MD USA). Mouse ZC3H12D was PCR amplified using primer sets 5'-GGTACCATGGAGCATCGGAGCAAGATGG-3' (SEQ ID NO: 7) and 5'-CTCGAGTTAAGGATCCCCCAACGGAGCACC-3' (SEQ ID NO: 8), followed by pCRBluntll vector (Thermo) for cloning. The cloned fragments were double-strand digested by Kpnl-Xhol and subcloned with pcDNA3 with a C-terminal FLAG-tag attached. In order to establish a cell line stably expressing human ZC3H12D, one of the above constructs was transfected into the cell line and the cells were cultured in the presence of 400 µg/mL G418 for more than two weeks. After G418 selection, cell lysates were tested by Western blotting using a DDDDK antibody (MBL Co., Ltd, Japan) probe to confirm expression of the ZC3H12D-FLAG protein.

### (7) Immunohistochemistry and Cell Counting

Anti-ZC3H12D (ab1000862, Abcam) antibody was used to stain CD56+CD3-NK cells. The number of immunostained cell regions is shown as the number of pixels normalized to the DAPI signal. Labeled cells were detected by confocal or fluorescence microscopy and normalized to total surface area.

### Embodiment 1

### Synthesis of Chemically Modified RNA

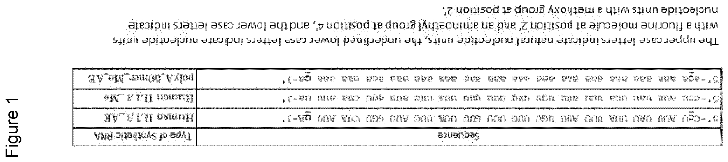

Two types of RNA (Human IL1β_AE and Human IL1β_Me) with the base sequence shown in Figure 1 (partial sequence of human IL1β (SEQ ID NO: 6)) and chemical modifications were synthesized according to the usual method. A nucleotide unit having a fluorine atom at the 2' position and an aminoethyl group at the 4' position was synthesized over its entire length according to the phosphoramidite method in accordance with JP 2019-10035 A. A 50-mer PolyA (PolyA_50mer_Me_AE) (SEQ ID NO: 9) with the chemical modification shown in Figure 1 was also synthesized as a control.

### Embodiment 2

### Confirmation of Cell Migration Ability Activation by Chemically Modified RNA in ZC3H12D- Overexpressing Cells

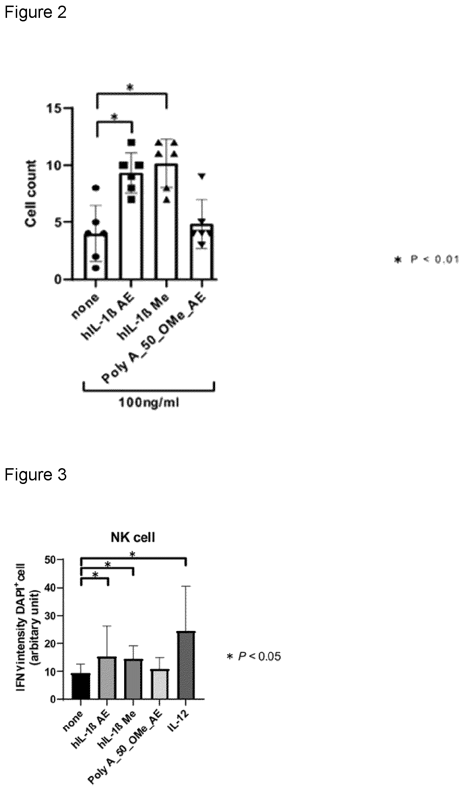

The two chemically modified types of RNA synthesized in Embodiment 1 and the 50-mer polA_50mer_Me_AE serving as a control were administered in a medium containing 786-O cells overexpressing ZC3H12D, and cell migration ability was measured by the average number of migrated cells. The concentration of RNA was 100 ng/mL. Results are shown in Figure 2.

As shown in Figure 2, the two types of synthetic RNA significantly enhanced migration activity versus no addition and control.

### Embodiment 3

### Confirmation of IFNγ Induction by Chemically Modified RNA in Human NK Cells

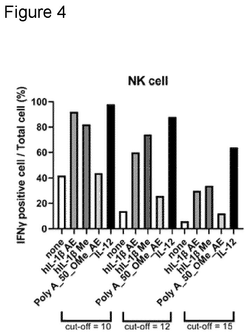

The effect of chemically modified RNA on IFNγ production in human CD56⁺CD3⁻NK cells was evaluated using the two types of chemically modified RNA synthesized in Embodiment 1 and the 50-mer polyA_50mer_Me_AE serving as a control. The results are shown in Figure 3 and Figure 4.

Figure 3 shows the signal intensity obtained from INFγ by DAPI staining. As shown in Figure 3, both of the two types of chemically modified RNA produced significantly more INFγ compared to when neither was added.

Figure 4 shows the number of INFγ positive NK cells per total NK cells. As shown in Figure 4, the two chemically modified types of RNA exhibited high INFγ-producing cell counts.

In summary, the RNA of the present disclosure, although partially chemically modified, had migration activating ability and INFγ-inducing activating ability.

### Embodiment 4

### Synthesis of Chemically Modified RNA

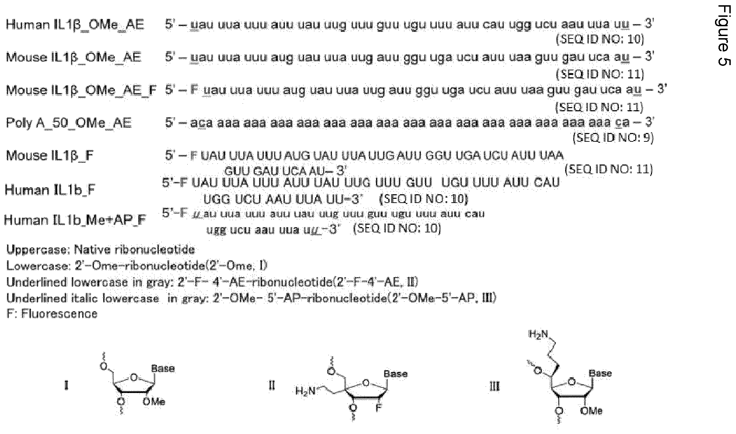

Seven types of RNA (Human IL1β_OME_AE, MouseIL1β_OMe_AE, MouseIL1β_OMe_AE_F, MouseIL1β_F, Human IL1β_OME_AP, Human IL1β_OME_AP_F, Human IL1β_F) with the sequence shown in Figure 5 (partial sequence of human IL1β (SEQ ID NO: 10), partial mouse IL1β sequence (SEQ ID NO: 11)), and chemical modifications were synthesized according to the usual method. A nucleotide unit having a fluorine atom at the 2' position and an aminoethyl group at the 4' position was synthesized over its entire length using the phosphoramidite method in accordance with JP 2019-10035 A. A nucleotide unit having a methoxy group at the 2' position and an aminopropyl group at the 5' position was synthesized over its entire length using the phosphoramidite method in accordance with WO 2019/088179 A1. A 50-mer PolyA (PolyA_50_OMe_AE) (SEQ ID NO: 9) with chemical modifications shown in Figure 5 was also synthesized as a control. MouseIL1β_OMe_AE_F, MouseIL1β_F, Human IL1β_OME_AP_F and Human IL1β_F were labeled with fluorescein at the 5' end.

### Embodiment 5

### Evaluation of RNase Resistance of Chemically Modified RNA

Using the fluorescein-labeled MouseIL1β_OME_AE_F and MouseIL1β_F synthesized in Example 4, synthetic RNA having, at each end, a nucleotide unit (2'-F-4'-AE) with a fluorine atom at the 2' position and an aminoethyl group at the 4' position and having all other nucleotide units contain a methoxy group at the 2' position (2'-OMe), was evaluated for nuclease (RNase) resistance in the serum of carcinoma-bearing mice. Carcinoma-bearing mice were produced by injecting 1 × 10⁶ E0771 cells/mouse into the mammary glands of 8- to 12-week-old C57BL/6 mice. Carcinoma-bearing mouse serum was obtained by intracardiac blood collection from mice that had formed a mass of approximately 1 cm or less in the mammary gland one week after injection of E0771 cells.

Using the fluorescein-labeled Human IL1β_OMe_AP_F and Human IL1β_F synthesized in Example 4, synthetic RNA having, at each end, a nucleotide unit (2'-OMe-5'-AP) with a methoxy group at the 2' position and an aminopropyl group at the 5' position and having all other nucleotide units (2'-OMe) contain a methoxy group at the 2' position (2'-OMe), was evaluated for nuclease (RNase) resistance in bovine serum.

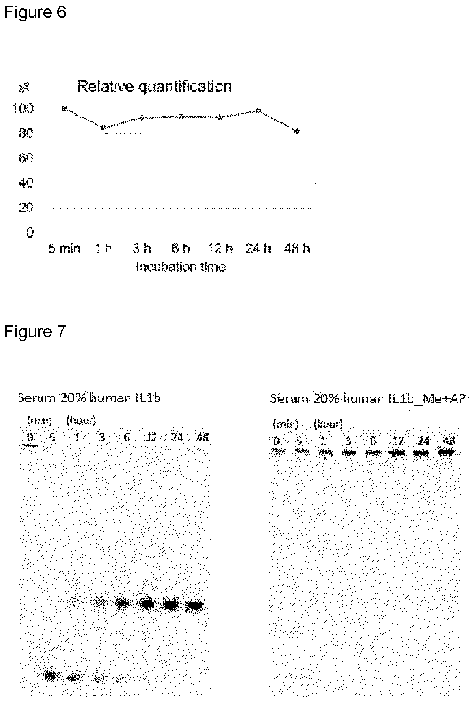

For nuclease resistance, 300 pmol of each RNA was dissolved in 37.5 µL of OPTI-MEM, from which 1.1 µL was aliquoted, and 15 µL of loading buffer (10 m MEDTA, 90% formamide) was added to obtain 0 min samples. Next, 4 µL (10%) of carcinoma-bearing mouse serum or 4 µL (10% and 20%) and 9.1 µL (20%) of bovine serum were added and the samples incubated at 37°C. After 5 minutes, 1 hour, 3 hours, 6 hours, 12 hours, 24 hours, and 48 hours, the reaction was stopped by adding 2 µL of the reaction solution to 10 µL of loading solution dispensed beforehand into separate sampling tubes. The reaction solution was electrophoresed with 20% denaturing polyacrylamide gel (PAGE) (500V, 20mA) and analyzed using Image analyzer LAS-4000 (Fujifilm Corporation). The results are shown in FIG. 6 and FIG. 7. In Figure 6, the amount of RNA (%) after 5 min to 48 hrs is shown relative to the amount of RNA at 0 min based on the electrophoresis results.

Denaturing PAGE was prepared using the following method. First, 40% acrylamide (19:1) solution (20 ml), urea (16.8 g), and 10 × TBE buffer (4 ml) were added, the contents were dissolved, and H₂O was added to make 40 ml. Finally, APS (27.5 mg) was added and dissolved, TEMED (20 µL) was added. The resulting solution was shaken, poured between two glass plates fixed with a spacer in between, and allowed to stand for at least 1 hour to solidify. Note that 1 × TBE buffer was used as the buffer for electrophoresis.

As shown in Figure 6, the chemically modified RNA with one nucleotide unit (2'-F-4'-AE) at each end and the rest being nucleotide unit (2'-OMe) remained at 80% RNA content even after 48 hours in serum. In contrast, in the case of RNA in which all nucleotide units were natural nucleotide units, all of the RNA had usually disappeared after 5 minutes. These results indicate that RNA with (2'-F-4'-AE) at the ends has superior resistance to RNase in serum.

As shown in Fig. 7, the chemically modified RNA with one nucleotide unit ((2'-OMe-5'-AP) at each end and the rest being nucleotide unit (2'-OMe) remained at almost 100% RNA content even after 48 hours in 20% serum. In contrast, in the case of RNA in which all nucleotide units were natural nucleotide units, all of the RNA had usually disappeared after 5 minutes. These results indicate that RNA with (2'-OMe-5'-AP) at the ends has superior resistance to RNase in serum.

### Embodiment 6

### Confirmation of Cell Migration Ability Activation by Chemically Modified RNA

Human IL1β_OME_AE synthesized in Example 4 and PolyA_50_OMe_AE serving as a control were administered via filters into cell suspensions containing 786-O cells overexpressing ZC3H12D protein and the number of migrated cells was counted. A Boyden chamber (NeuroProbe) was used as the device. The upper and lower wells were separated by a polycarbonate filter (Nucleopore; Costar) with a pore size of 5 µm. The upper wells were filled with cell suspension (1 × 10⁶/ml) and the lower wells with medium containing each of the types of RNA at 1 ng/ml, 10 ng/ml, 100 ng/ml and 1 µg/ml concentrations. This was incubated for three hours and the number of cells migrating to the lower wells was counted. The results are shown in Table 1.

**Table 1**

| | none | IL1β_OMe_AE | Poly A_OMe_AE |
|---|---|---|---|
| Number of migrated cells | 100 | 150 | 100 |

As shown in Table 1, when Human IL1β_OME_AE was administered, the number of migrating cells averaged 150 at each concentration of RNA, with the number of migrating cells without adding RNA set at 100. The number generally increased significantly with RNA concentration, reaching about 200 at 100 ng/ml. Meanwhile, when PolyA_50_OMe_AE was administered, the results were similar at every concentration to when RNA is not added, with an average of 110. In summary, Human IL1β_OME_AE with one (2'-F-4'-AE) each at both ends contributes to an increase in the migratory ability of NK cells expressing the ZC3H12D protein.

### Embodiment 7

### Confirmation of IFNγ Induction

The effect of chemically modified RNA on IFNγ production in human CD56⁺CD3⁻NK cells was evaluated using Human IL1β_OME_AE synthesized in Example 4 and PolyA_50_OMe_AE used as a control. Human CD56⁺CD3⁻NK cells were harvested by FACS from cryopreserved human PBMCs. Twenty-four hours after priming with 10 ng/mL of each RNA in HPLM medium (Gibco) in the presence of 200 U/mL IL-2 (BioLegend), the cells were stained with anti-human IFN-γ antibody (BioLegend). IL-12 (Proteintech) was used as a positive control for IFNγ induction. The results are shown in Table 2.

**Table 2**

| | none | IL1β_OMe_AE | Poly A_OMe_AE | IL-12 |
|---|---|---|---|---|
| Number of IFNγ⁺ cells | 100 | 230 | 110 | 230 |

As shown in Table 2, Human IL1β_OME_AE induced a 2.3-fold higher level of INFγ than IFNγ without addition. This was comparable to the positive control. Meanwhile, when PolyA_50_OMe_AE was used, IFNγ was induced only to the same extent as without addition. In summary, Human IL1β_OME_AE with one (2'-F-4'-AE) each at both ends contributes to the increased production of INFγ by human NK cells.

### Example 8

### Evaluation of Human NK Cells Killing Ability

Using the Zombie Green Fixable Viability Kit (BioLegend), the killing ability of human CD56⁺CD3⁻NK cells was evaluated for Human IL1β_OME_AE synthesized in Example 4 and PolyA_50_OMe_AE serving as a control. Specifically, 24 hours after 1:1 co-culturing of human CD56⁺CD3⁻NK cells primed with 10 ng/mL of each RNA for 18 hours and cancer cells (Caco-2: colon cancer cell line), the percentage of tumor cells stained dark green was observed. The results are shown in Table 3.

**Table 3**

| | none | IL1β_OMe_AE | Poly A_OMe_AE | IL-12 |
|---|---|---|---|---|
| Number of Zombie ⁺ cells | 100 | 250 | 120 | 350 |

As shown in Table 3, Human IL1β_OME_AE induced a 2.5-fold higher level of killed cells than without addition. This was comparable to the positive control. Meanwhile, when PolyA_50_OMe_AE was used, the level of killed cells was similar to the level without addition. These results indicate that Human IL1β_OME_AE, which has one (2'-F-4'-AE) at each end, contributes to the increased killing ability of human NK cells.

### Embodiment 9

### Evaluation of Anti-Metastatic Activity in Pseudo-Lung Metastasis Mouse Model

Human IL1β_OME_AE synthesized in Embodiment 4 and PolyA_50_OMe_AE serving as a control were evaluated for their antimetastatic activity. Each RNA (Human IL1β_OME_AE and PolyA_50_OMe_AE, 1 µg each) and PBS serving as a negative control were injected every few days via the tail vein of mice that were carcinoma-bearing with E0771 breast cancer cells. Forty-eight hours after injection of tumor cells (E0771 breast cancer cells) stained with PKH-26 (Sigma-Aldrich) via the tail vein, the mice were dissected and the number of PKH-26 positive cells in the lungs was observed. The results are shown in Table 4.

**Table 4**

| | PBS | IL1β_OMe_AE | Poly A_OMe_AE |
|---|---|---|---|
| Number of PKH-26⁺ cells | 100 | 65 | 95 |

As shown in Table 4, 65% positive cell counts were observed with Human IL1β_OME_AE compared to when PBS was added. Meanwhile, when PolyA_50_OMe_AE was used, positive cells were observed at the same level as when PBS was added. In summary, Human IL1β_OME_AE with one (2'-F-4'-AE) each at both ends contributes to the inhibition of tumor cell metastasis in vivo.

### SEQUENCE LIST FREE TEXT

SEQ ID NOS. 7, 8: Primer
SEQ ID NO. 9: Control RNA

## Claims

1. An antitumor agent containing, as an active ingredient, RNA having a base sequence selected from the group consisting of (1) and (2) below, and having at least one first nucleotide unit represented by Formula (1) below or at least one second nucleotide unit represented by Formula (2) below in a 3' end region within 20 bases of the 3' end thereof and/or in a 5' end region within 25 bases of the 5' end thereof.
(1) A base sequence represented by SEQ ID NO: 2 or a base sequence having 90% or more identity with such sequence
(2) A base sequence consisting of between 20 and 200 consecutive nucleotides of the base sequence described in (1) above 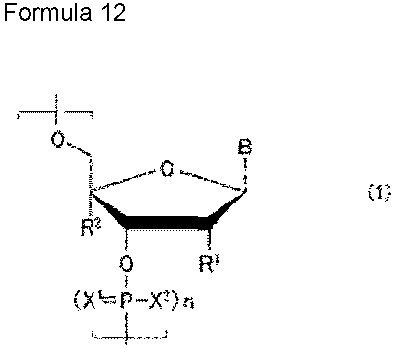 (In Formula (1), R¹ represents a hydroxyl group, a hydroxyl group with a hydrogen atom substituted with an alkyl or alkenyl group, or a halogen atom; R² represents NHR³ with a linking group, the linking group being a divalent hydrocarbon group with one or more carbon atoms; R³ represents a hydrogen atom, an alkyl group, or an alkenyl group; n is 0 or 1; X¹ represents an oxygen atom or sulfur atom; X² represents OH (or O⁻) or SH (or S⁻); and B represents a purine base or a pyrimidine base.) 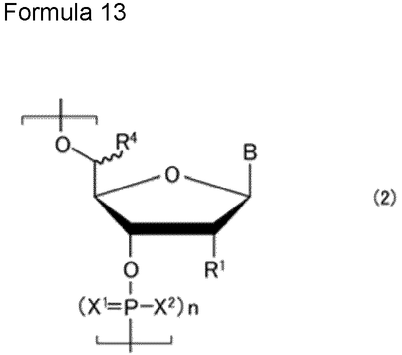 (In Formula (2), R¹ represents a hydroxyl group, a hydroxyl group with a hydrogen atom substituted with an alkyl or alkenyl group, or a halogen atom; R⁴ represents NHR⁵ with a linking group, the linking group being a divalent hydrocarbon group with one or more carbon atoms; R⁵ represents a hydrogen atom, an alkyl group, or an alkenyl group; n is 0 or 1; X¹ represents an oxygen atom or sulfur atom; X² represents OH (or O⁻) or SH (or S⁻); and B represents a purine base or a pyrimidine base.)

2. The antitumor agent according to claim 1, wherein the RNA has between 1 and 20 first nucleotide units or second nucleotide units per region in the 3' end region and/or 5' end region.

3. The antitumor agent according to claim 1, wherein the first nucleotide unit or second nucleotide unit is a nucleotide unit wherein R¹ is a methoxy group or a fluorine atom and R² is an aminoethyl group in Formula (1) above.

4. The antitumor agent according to claim 1, wherein the first nucleotide unit is a nucleotide unit in which n is 1, R¹ is a fluorine atom, and R² is an aminoethyl group, and the antitumor agent has at least one first nucleotide unit within two bases from the 3' end of the 3' end region and at least one first nucleotide unit within two bases from the 5' end of the 5' end region.

5. The antitumor agent according to claim 1, wherein the second nucleotide unit is a nucleotide unit in which n is 1, R¹ is a methoxy group, and R⁴ is an aminopropyl group, and the antitumor agent has at least one second nucleotide unit within two bases from the 3' end of the 3' end region and at least one second nucleotide unit within two bases from the 5' end of the 5' end region.

6. The antitumor agent according to claim 4 or 5, wherein the base sequence has 90% or more identity with between 30 and 70 consecutive bases of the base sequence represented by SEQ ID NO: 4.

7. The antitumor agent according to claim 4 or 5, wherein the base sequence has 90% or more identity with between 40 and 60 consecutive bases of the base sequence represented by SEQ ID NO: 4.

8. The antitumor agent according to claim 4 or 5, wherein the base sequence is a base sequence having 90% or more identity with the base sequence represented by SEQ ID NOS: 6 or 10.

9. The antitumor agent according to claim 8, wherein a base in the nucleotide unit is cytosine or uracil.

10. The antitumor agent according to claim 1, wherein n is 1 in the first or second nucleotide unit, the RNA has a first or second nucleotide unit in the 3' end region and/or 5' end region in which either X¹ or X² in Formula (1) above is S or SH(S⁻).

11. The antitumor agent according to claim 1, wherein at least 80% of the total number of uridine nucleosides in the RNA contain uridine analogues are selected from the group consisting of pseudouridine, 1-methylpseudouridine, 1-ethylpseudouridine, 2-thiouridine, 4'-thiouridine, 5-methylcytosine, 2-thio-1-methyl-1-deaza-pseudouridine, 2-thio-1-methylpseudouridine, 2-thio-5-aza-uridine, 2-thio-dihydropseudouridine, 2-thio-dihydropseudouridine, 2-thio-pseudouridine, 4-methoxy-2-thio-pseudouridine, 4-methoxy-pseudouridine, 4-thio-1-methyl-pseudouridine, 4-thio-pseudouridine, 5-aza-uridine, dihydropseudouridine, 5-methoxyuridine, and 2'-O-methyluridine.

12. The antitumor agent according to claim 1, wherein the RNA is a variant of RNA having the base sequence represented by SEQ ID NO: 1, and the coding sequence from the 88th to 897th base in the base sequence represented by SEQ ID NO: 1 has bases substituted to have 1 to 5 additional stop codons compared to the natural coding sequence.

13. The antitumor agent according to claim 1, wherein the length of the RNA is 20 to 100 bases long.

14. An immunostimulant containing, as an active ingredient, RNA having a base sequence selected from the group consisting of (1) and (2) below, and having at least one first nucleotide unit represented by Formula (1) below or at least one second nucleotide unit represented by Formula (2) below in a 3' end region within 20 bases of the 3' end thereof and/or in a 5' end region within 25 bases of the 5' end thereof.
(1) A base sequence represented by SEQ ID NO: 2 or a base sequence having 90% or more identity with such sequence
(2) A base sequence consisting of between 20 and 200 consecutive nucleotides of the base sequence described in (1) above 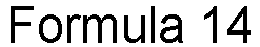 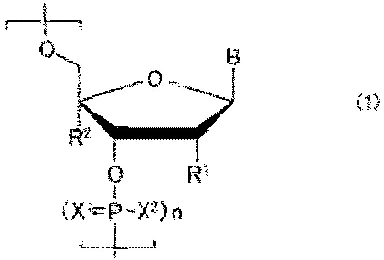 (In Formula (1), R¹ represents a hydroxyl group, a hydroxyl group with a hydrogen atom substituted with an alkyl or alkenyl group, or a halogen atom; R² represents NHR³ with a linking group, the linking group being a divalent hydrocarbon group with one or more carbon atoms; R³ represents a hydrogen atom, an alkyl group, or an alkenyl group; n is 0 or 1; X¹ represents an oxygen atom or sulfur atom; X² represents OH (or O⁻) or SH (or S⁻); and B represents a purine base or a pyrimidine base.) 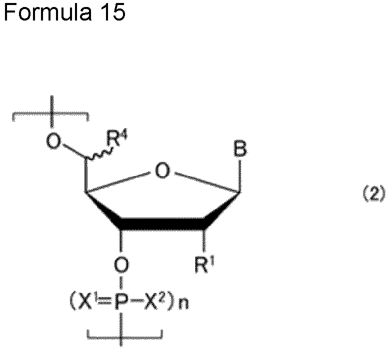 (In Formula (2), R¹ represents a hydroxyl group, a hydroxyl group with a hydrogen atom substituted with an alkyl or alkenyl group, or a halogen atom; R⁴ represents NHR⁵ with a linking group, the linking group being a divalent hydrocarbon group with one or more carbon atoms; R⁵ represents a hydrogen atom, an alkyl group, or an alkenyl group; n is 0 or 1; X¹ represents an oxygen atom or sulfur atom; X² represents OH (or O⁻) or SH (or S⁻); and B represents a purine base or a pyrimidine base.)

15. A tumor metastasis inhibitor containing, as an active ingredient, RNA having a base sequence selected from the group consisting of (1) and (2) below, and having at least one first nucleotide unit represented by Formula (1) below or at least one second nucleotide unit represented by Formula (2) below in a 3' end region within 20 bases of the 3' end thereof and/or in a 5' end region within 25 bases of the 5' end thereof.
(1) A base sequence represented by SEQ ID NO: 2 or a base sequence having 90% or more identity with such sequence
(2) A base sequence consisting of between 20 and 200 consecutive nucleotides of the base sequence described in (1) above 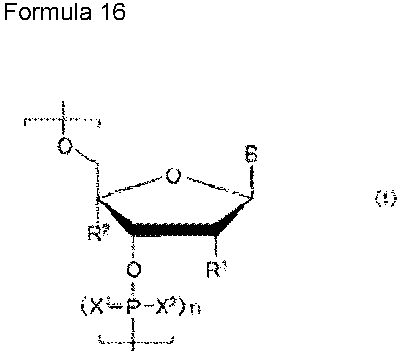 (In Formula (1), R¹ represents a hydroxyl group, a hydroxyl group with a hydrogen atom substituted with an alkyl or alkenyl group, or a halogen atom; R² represents NHR³ with a linking group, the linking group being a divalent hydrocarbon group with one or more carbon atoms; R³ represents a hydrogen atom, an alkyl group, or an alkenyl group; n is 0 or 1; X¹ represents an oxygen atom or sulfur atom; X² represents OH (or O⁻) or SH (or S⁻); and B represents a purine base or a pyrimidine base.) 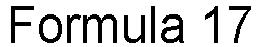 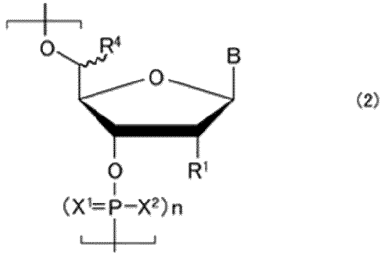 (In Formula (2), R¹ represents a hydroxyl group, a hydroxyl group with a hydrogen atom substituted with an alkyl or alkenyl group, or a halogen atom; R⁴ represents NHR⁵ with a linking group, the linking group being a divalent hydrocarbon group with one or more carbon atoms; R⁵ represents a hydrogen atom, an alkyl group, or an alkenyl group; n is 0 or 1; X¹ represents an oxygen atom or sulfur atom; X² represents OH (or O⁻) or SH (or S⁻); and B represents a purine base or a pyrimidine base.)

16. An enhancer of an immunostimulatory effect of an immunostimulant or an antitumor effect of an antitumor agent, the enhancer containing, as an active ingredient, RNA having a base sequence selected from the group consisting of (1) and (2) below, and having at least one first nucleotide unit represented by Formula (1) below or at least one second nucleotide unit represented by Formula (2) below in a 3' end region within 20 bases of the 3' end thereof and/or in a 5' end region within 25 bases of the 5' end thereof.
(1) A base sequence represented by SEQ ID NO: 2 or a base sequence having 90% or more identity with such sequence
(2) A base sequence consisting of between 20 and 200 consecutive nucleotides of the base sequence described in (1) above 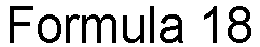 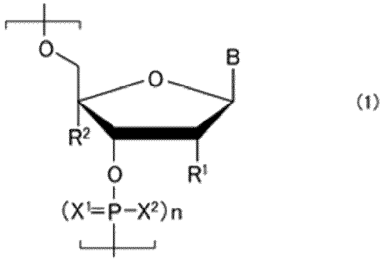 (In Formula (1), R¹ represents a hydroxyl group, a hydroxyl group with a hydrogen atom substituted with an alkyl or alkenyl group, or a halogen atom; R² represents NHR³ with a linking group, the linking group being a divalent hydrocarbon group with one or more carbon atoms; R³ represents a hydrogen atom, an alkyl group, or an alkenyl group; n is 0 or 1; X¹ represents an oxygen atom or sulfur atom; X² represents OH (or O⁻) or SH (or S⁻); and B represents a purine base or a pyrimidine base.) 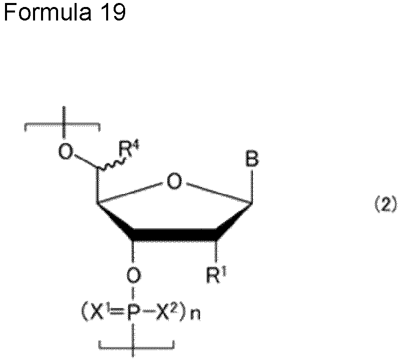 (In Formula (2), R¹ represents a hydroxyl group, a hydroxyl group with a hydrogen atom substituted with an alkyl or alkenyl group, or a halogen atom; R⁴ represents NHR⁵ with a linking group, the linking group being a divalent hydrocarbon group with one or more carbon atoms; R⁵ represents a hydrogen atom, an alkyl group, or an alkenyl group; n is 0 or 1; X¹ represents an oxygen atom or sulfur atom; X² represents OH (or O⁻) or SH (or S⁻); and B represents a purine base or a pyrimidine base.)
